# EUROPEAN PATENT APPLICATION

(11) **EP 1 844 743 A2**
(43) Date of publication of application: **17.10.2007**
(21) Application number: 07007539.5
(22) Date of filing: 12.04.2007
(51) Int. Cl.: A61F 5/56

(54) **Apparatus for preventing sleeping respiratory obstruction**

(30) Priority: 14.04.2006 KR 20060034042; 14.04.2006 KR 20060034043
(71) Applicant: Bio Sleep Med Co., Ltd., Seongbuk-gu Seoul (KR)
(72) Inventor: Hong, Junghwa, Anyang-si, Gyeonggi-do (KR); Shin, Chol, Seocho-gu, Seoul (KR)
(74) Representative: Samson & Partner

(57) **Abstract**

An apparatus for preventing a sleeping respiratory obstruction includes a pillow sheet having a plurality of chambers on which a user's body is arranged for preventing a sleeping respiratory obstruction, and a control module for controlling inflation and deflation of the chambers in the pillow sheet by supplying and discharging pressure to and from the chambers, to thereby allow the pillow sheet to support a head and a neck of the user during sleep. The sleeping respiratory obstruction apparatus further includes a wearable unit detachably coupled to the pillow sheet and be worn on the human body during sleep.

## Description

The present invention relates to an apparatus for preventing a sleeping respiratory obstruction; and, more particularly, to an apparatus for preventing a sleeping respiratory obstruction, capable of preventing and treating an obstructive sleep apnea caused by repetitive closure of an upper airway in a neck of a human body and a habitual snoring related to the obstructive sleep apnea.

In general, a habitual snoring, an obstructive sleep apnea and an upper airway resistance syndrome classified as a sleeping respiratory obstruction are diseases in which the repetitive closure of the upper airway occurs during sleep. Such diseases hinder sound sleep by deteriorating sleep efficiency at night and especially decrease a blood oxygen saturation rate [see Chrokroverty S. (1994) Sleep Disorder Medicine. Butterworth-Heinemann].

The sleeping respiratory obstruction causes a daytime drowsiness, a deteriorated power of concentration, a failure of memory, a decreased learning ability, a chronic fatigue and the like. Further, the sleeping respiratory obstruction leads to accidents in industrial fields and workplaces and traffic accidents due to a drowsy driving, thereby inflicting social and economical damages.

In addition, there have been several reports on a close relationship between the sleeping respiratory obstruction and the occurrence of obesity, high blood pressure, diabetes, dementia, cardiovascular diseases, sexual function decline, cerebrovascular diseases, paralysis and metabolic syndrome [see Prospective study of the association between sleeping respiratory obstruction and hypertension. N Engl Med 2000; 342: 1378-1384].

The sleeping respiratory obstruction has been commonly observed both in men and women worldwide. In the U.S., 28 % of men (about 75 million men) and 16 % to 18 % of women (about 48 million women) suffer from the sleeping respiratory obstruction [see The occurrence of sleeping respiratory obstruction among middle-aged adults. N Engl J Med 1993; 328: 1230-1235].

According to a recent research in Korea, 27 % of Korean men (about 120 million men) and 16 % of Korean women (76 million women) suffer from the sleeping respiratory obstruction [Prevalence of sleeping respiratory obstruction in middle-aged Korean men and women. Am J Respir Crit Care Med. 2004; 170(10): 1108-13]. This indicates that two to three out of ten adults are experiencing the sleeping respiratory obstruction.

Although an obesity index and an abdominal circumference recognized as major factors of the sleeping respiratory obstruction in previous researches are comparatively lower for Koreans than for Americans, a prevalence rate of the sleeping respiratory obstruction in Korea is similar to that in the U.S. However, the obesity index and the abdominal circumference in Korea are expected to increase due to western style eating habits, so that the number of sleeping respiratory obstruction patients will grow.

As for the causes of the sleeping respiratory obstruction, there can be suggested obesity, family history, anatomical abnormal structure, gender difference (more common in men than in women), internal diseases (thyroid diseases) and the like.

Structurally, soft tissues including nasopharynx, oropharynx and hypopharynx close an upper airway serving as an air inlet/outlet passageway during sleep.

When a patient falls asleep, an electromyogram shows a great decrease in strength of muscles. The decrease in the muscle strength leads to a release of the soft tissues of the upper airway and reduced activities of respiratory muscles, thereby closing the upper airway during sleep [see Chokroverty S. (1994) Sleep Disorder Medicine. Butterworth-Heinemann] .

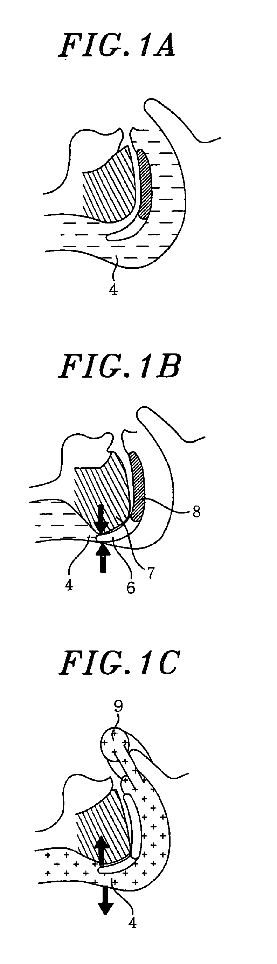

Figs. 1A to 1C illustrate an upper airway closure leading to a sleeping respiratory obstruction.

In general, a fluid flow rate Q is obtained by multiplying a flow path cross sectional area A by a fluid speed v, i.e., Q = A · v.

Such an equation can also be applied to a case where A, v and Q, respectively, indicate a cross sectional area of an upper airway into which air is introduced and discharged by breathing during sleep, an air speed and an air introduction/discharge amount. In case the air amount Q required for supplying oxygen to a human body is constant, a decrease of the cross sectional area of the upper airway leads to an increase of the air speed v, which causes a snoring.

When the upper airway is closed by the soft tissues, the cross sectional area A of the upper airway becomes zero and, also, the air inlet/outlet amount becomes zero, thereby causing an obstructive sleep apnea.

An upper airway 4 for introducing air into a bronchus and a lung (not shown) is sufficiently secured in a normal state shown in Fig. 1A. However, referring to an obstructive sleep apnea state illustrated in Fig. 1B, a soft tissue 6 extended from a back part of a pallet 8 is pressed by a self-weight and a weight of a tongue 7, thereby closing the upper airway 4.

Sleeping in a supine position worsens the closure of upper airway. Further, the closure of upper airway leads to the obstructive sleep apnea in which breathing stops or is disrupted during sleep.

Snoring occurs when the upper airway 4 is partially closed during sleep.

Various methods have been attempted to treat the snoring or the obstructive sleep apnea.

As for representative methods, there have been attempted a physical treatment for allowing air to smoothly pass through the upper airway 4 by adjusting a sleeping position to a lateral position or a prone position; a surgical treatment for removing flabby soft tissues 6 of the upper airway 4; and a non-surgical treatment for allowing the upper airway 4 to be constantly opened by applying a positive airway pressure to a patient through a continuous positive airway pressure (CPAP) mask 9 by being attached to a nose during sleep.

However, such treatments also have the following problems. The physical treatment may not constantly maintain the lateral position or the prone position. The surgical treatment has a high recurrence rate due to a regeneration of soft tissues. The non-surgical treatment shows low compliance with the treatment due to uncomfortableness of wearing the CPAP mask during sleep.

In order to overcome such problems, there has been suggested another method for treating an obstructive sleep apnea by using functional pillow sheets during sleep. Especially, there has been developed a pillow for correcting a body position by raising a lateral position or a head position.

However, the functional pillows that have been developed so far show no dramatic improvement [Elevated posture for management of obstructive sleep apnea. Sleep and breathing, 2004; 8(4): 193-200].

Recently, a memory foam pillow sheet made of polymer foam developed by NASA scientists has been commercially marketed as the pillow for preventing a sleeping respiratory obstruction. Although the memory foam pillow is an ergonomically designed pillow capable of absorbing load and shock transmitted to a human body and returning to an original shape after the load is released, it is ineffective for the treatment of a snoring or an obstructive sleep apnea.

This is because the pillow for treating a sleeping respiratory obstruction is not able to adjust a shape thereof according to structural changes in the upper airway 4 and a function thereof while taking into account a degree of the snoring and the obstructive sleep apnea.

Such a pillow cannot be actively controlled because it has been developed without considering the frequent changes of body position by patients experiencing the sleeping respiratory obstruction and characteristics of heads, cervical vertebrae and body position changes in different patients. Moreover, such a pillow serves as a device for changing a sleeping position rather than a device for providing a professional medical treatment.

Therefore, the pillow as set forth above is ineffective to treat or relieve the snoring and the obstructive and prevent a recurrence of the sleeping respiratory obstruction.

It is, therefore, a primary object of the present invention to provide an apparatus for preventing a sleeping respiratory obstruction, capable of maintaining a lateral position of a user during sleep and an optimal pressure distribution in heads and cervical vertebrae according to characteristics of heads and cervical vertebrae in different patients.

It is another object of the present invention to provide an apparatus for preventing a sleeping respiratory obstruction capable of detecting a snoring during sleep and capable of detecting a decreased blood oxygen saturation rate due to a snoring and an obstructive sleep apnea.

It is still another object of the present invention to provide an apparatus for preventing a sleeping respiratory obstruction capable of raising a chin and extending cervical vertebrae of a user, in case the snoring or the obstructive sleep apnea is detected, by automatically inflating neck supporting portions (a portion under the head and a portion under the neck) of the apparatus to reduce a flow resistance of an upper airway and prevent an airway obstruction caused by the head and the cervical vertebrae and, also capable of changing a sleeping position to a lateral position, in case the snoring or the obstructive sleep apnea has not been treated in spite of the inflation of the neck supporting portions of the apparatus.

In accordance with one aspect of the present invention, there is provided an apparatus for preventing a sleeping respiratory obstruction, includes: a pillow sheet having a plurality of chambers on which a body of a user is supported; and a control module for controlling inflation and deflation of the chambers supporting the body of the user in order to open an upper airway of the user being subjected to the sleep respiratory obstruction during sleep.

In accordance with a second aspect of the present invention, there is provided an apparatus for preventing a sleeping respiratory obstruction, includes: a pillow sheet having a plurality of chambers on which a body of a user is arranged; a pressure controller for supplying and discharging pressure to and from the respective chambers for an inflation and a deflation of the respective chambers; a pressure detection unit for detecting pressure in each of the chambers; a storage unit for storing optimal pressure pattern data obtained by making use of patterns of pressure changes in each of the chambers during normal sleep; and an artificial intelligence controller for comparing the pressure pattern data with the pressure in each of the chambers received from the pressure detection unit to check whether or not a respiratory obstruction has occurred, and providing to the pressure controller, in case the occurrence of the respiratory obstruction has been detected, pressure control signal for controlling the inflation and the deflation of the chambers so that an upper airway of the user is made open.

In accordance with a third aspect of the present invention, there is provided an apparatus for preventing a sleeping respiratory obstruction, includes: a pillow sheet having a plurality of chambers on which a body of a user is arranged; a wearable unit detachably coupled to the pillow sheet and be worn on the body during sleep; a pressure controller for supplying and discharging pressure to and from the respective chambers for an inflation and a deflation of the respective chambers; a pressure detection unit for detecting pressure in each of the chambers; a storage unit for storing optimal pressure pattern data obtained by making use of patterns of pressure changes in each of the chambers during normal sleep; and an artificial intelligence controller for comparing the pressure pattern data with the pressure in each of the chambers received from the pressure detection unit to check whether or not a respiratory obstruction has occurred, and providing to the pressure controller, in case the occurrence of the respiratory obstruction has been detected, pressure control signal for controlling the inflation and the deflation of the chambers to support a head and a neck of the user so that an upper airway of the user is made open to thereby treat the respiratory obstruction.

The above and other objects and features of the present invention will become apparent from the following description of embodiments, given in conjunction with the accompanying drawings, in which:
Figs. 1A to 1C are exemplary diagrams illustrating a closure of an upper airway;
Figs. 2A and 2B show side cross-sectional views of a human body supported by an apparatus for preventing a sleeping respiratory obstruction in accordance with a first embodiment of the present invention;
Fig. 3 depicts a block diagram of the sleeping respiratory obstruction prevention apparatus shown in Figs. 2A and 2B;
Figs. 4A and 4B provide perspective views of a pillow sheet of the sleeping respiratory obstruction prevention apparatus in Figs. 2A and 2B;
Figs. 5A to 5D present top views and side views of a modified example of the pillow sheet shown in Fig. 3;
Figs. 6A to 6C present graphs showing breathing patterns;
Figs. 7A and 7B represent side views of a modified example of a chamber for treating a sleeping respiratory obstruction;
Figs. 8A and 8B describe perspective views of air cells;
Figs. 9A and 9B provide examples of arranging the air cell in the pillow sheet in accordance with the present invention;
Figs. 10A to 10C offer graphs illustrating pressure patterns in chambers in a supine position and a lateral position and conceptual diagrams of a chamber for returning a sleeping position of a human body to the supine position;
Fig. 11 offers a block diagram of an apparatus for preventing a sleeping respiratory obstruction in accordance with a second embodiment of the present invention;
Figs. 12A and 12B show an operation state of using the sleeping respiratory obstruction prevention apparatus shown in Fig. 11;
Figs. 13A to 13C provide a front view and rear views of the wearable unit shown in Fig. 11, respectively; and
Figs. 14A and 14B describe a front view and a rear view of another example of the wearable unit shown in Fig. 11, respectively.

Embodiments of the present invention will be described in detail with reference to the accompanying drawings.

### (First Embodiment)

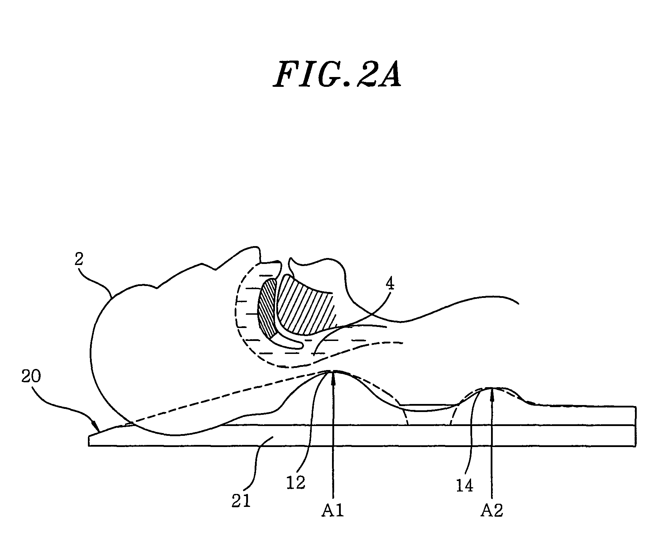

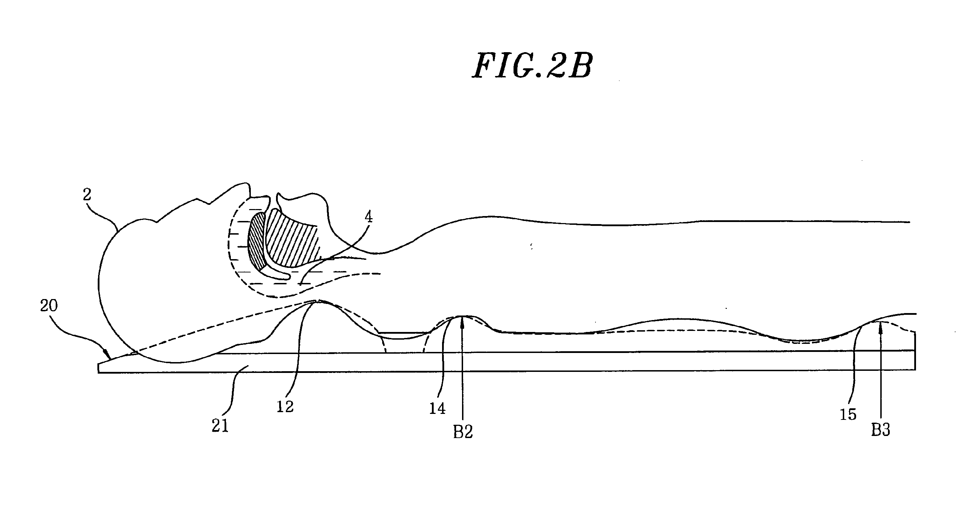

Figs. 2A and 2B show side cross-sectional views of a human body supported by an apparatus for preventing a sleeping respiratory obstruction having a pillow sheet 20 in accordance with a first embodiment of the present invention.

As shown in Figs. 2A and 2B, an upper airway 4 related to the sleeping respiratory obstruction is closed to a minimum level or fully opened by raising specific portions of a human body kept in a supine position during sleep. When the upper airway 4 is neither closed to a minimum level nor fully opened even by raising the specific portions, it can be closed to a minimum level or fully opened by changing the sleeping position to a lateral position.

To be specific, the upper airway 4 is opened by extending a curvature of cervical vertebrae (not shown) by way of raising a portion under the head in a direction of A1 in Fig. 2A, so that the sleeping respiratory obstruction can be treated. Moreover, the upper airway 4 is further opened by raising scapulae (not shown) by way of raising a portion under the neck in a direction of A2, so that the sleeping respiratory obstruction can be treated.

Further, when the sleeping respiratory obstruction such as a snoring, an obstructive sleep apnea or the like is not treated even by extending the curvature of the cervical vertebrae in the directions of Al and A2, as illustrated in Fig. 2B, the pillow sheet 20 extended to thighs of a human body is provided to prevent the sleeping respiratory obstruction. Then, only one longitudinal end portion of the pillow sheet 20 is raised in order to guide a sleeping position to a lateral position. As a result, the upper airway 3 is opened, and the sleeping respiratory obstruction is treated.

To be more specific, as can be seen from Fig. 2B, only one side of a lower neck portion 14 is raised by elevating in a direction of B2 one longitudinal end of the pillow sheet, the longitudinal end corresponding to the lower neck portion 14. Further, only one side of a thigh portion 15 is raised by elevating in a direction of B3 one longitudinal end of the pillow sheet, the longitudinal end corresponding to the thigh portion 15. Accordingly, the longitudinal ends of the pillow sheet for preventing a sleeping respiratory obstruction, which correspond to the lower neck portion 14 and the thigh portion 15, are inclined and, then, the sleeping position is changed to the lateral position. As a result, the upper airway 4 is opened, and the sleeping respiratory obstruction is treated.

In such a case, the longitudinal ends corresponding to the lower neck portion 14 and the thigh portion 15 of the pillow sheet need to be raised from the same side in order to change the sleeping position to the lateral position.

When the sleeping position is guided to the lateral position, it can be changed to either a right lateral position or a left lateral position.

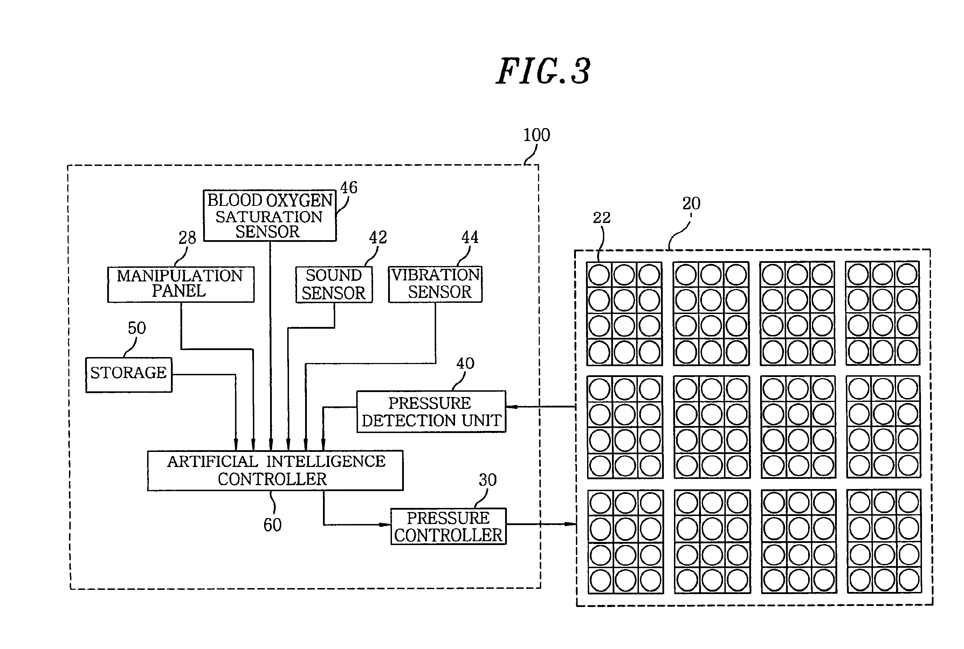

Referring now to Fig. 3, there is shown the sleeping respiratory obstruction apparatus in accordance with the present invention. The sleeping respiratory obstruction apparatus includes a pillow sheet 20 having a plurality of chambers 22.

The sleeping respiratory obstruction apparatus further includes a control module 100, which includes a pressure controller 30 for supplying and discharging pressure to and from the chambers 22 for an inflation and a deflation; a pressure detection unit 40 for detecting pressure in each of the chambers 22; a storage unit 50 for storing therein control data containing optimal pressure pattern data obtained by making use of patterns of pressure changes in each of the chambers 22 during normal sleep; and an artificial intelligence controller 60 for loading the control data, checking whether or not a respiratory obstruction has occurred by comparing the loaded control data with the pressure in each of the chambers 22 received from the pressure detection unit 40, and outputting to the pressure controller 30, in case the occurrence of the respiratory obstruction has been checked, pressure control signal for controlling the inflation and the deflation of the chambers 22 to thereby treat the respiratory obstruction by opening the upper airway.

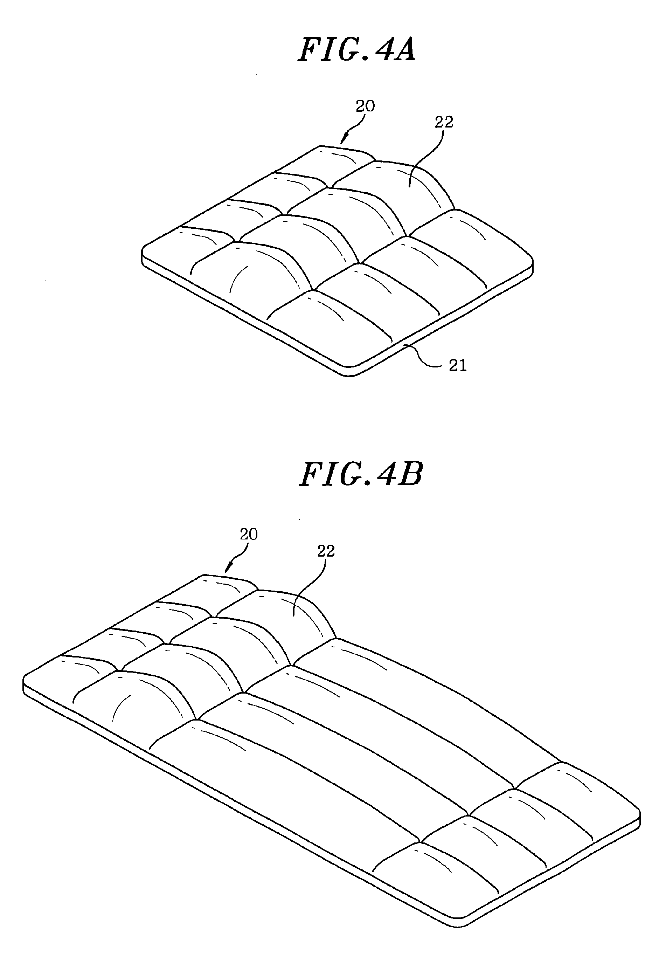

The pillow sheet 20 is formed to support a head 2, a lower head portion 12 and the lower neck portion 14, as shown in Fig. 2A or 4A, or formed to support the head 2, the lower head portion 12, the lower neck portion 14 and the thigh portion 15, as illustrated in Fig. 2B or 4B, to thereby allow a user to sleep in a proper position. The pillow sheet may have a cover (not shown) thereon to be felt comfortable by the human body.

Preferably, the pillow sheet 20 is divided into a plurality of chambers 22, which are symmetrically formed, as shown in Fig. 3.

For example, as exemplarily illustrated in Fig. 4A, the pillow sheet 20 may have twelve chambers 22 which are formed, for example, in three rows and four columns. Further, as illustrated in Fig. 4B, the pillow sheet 20 may have sixteen chambers 22 which are formed, for example, in four rows and four columns. However, it will be apparent to those skilled in the art that the chambers 22 may be arranged in various formats without being limited to the aforementioned examples.

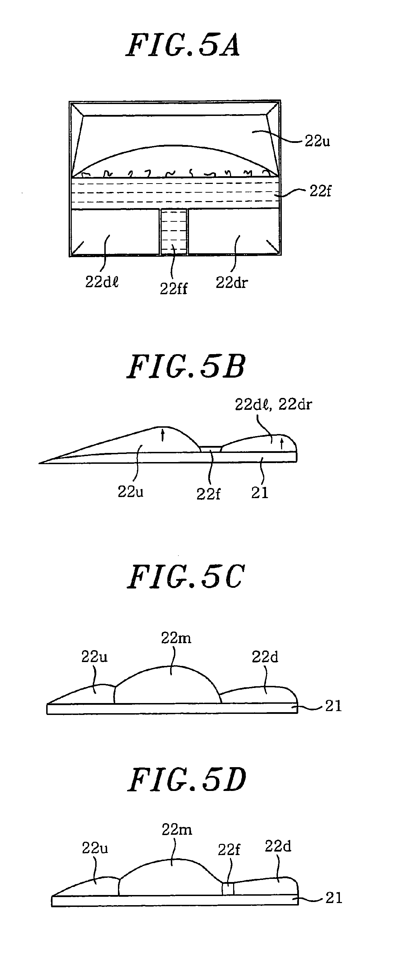

Alternatively, as illustrated in Fig. 5A and 5B which illustrates a top view and a side view of a modified example of the pillow sheet shown in Fig. 3, the pillow sheet may be formed with a longitudinal upper chamber 22u contacted with an upper portion of the head 2 of a user and a lower chamber having a right and a left lower chamber 22dr and 22dl contacted with a neck portion of the user. At this time, a fixing unit 22f may be horizontally provided between the upper chamber 22u and the lower chambers 22dr and 22dl. Further, another fixing unit 22f may be provided between the right lower chamber 22dr and the left lower chamber 22d1. The fixing units 22f and 22ff are maintained to be flat without being inflated even when pressure is supplied to each of the chambers 22.

Besides, as depicted in Fig. 5C, the pillow sheet may be formed with a single upper chamber 22u contacted with an upper portion of the head 2 of a user, a single intermediate chamber 22m contacted with a neck portion thereof and a lower chamber 22d contacted with a lower neck portion thereof.

In such a case, as shown in Fig. 5D, the intermediate chamber 22m and the lower chamber 22d may have therebetween a fixing unit 22f for lengthening a distance therebetween.

Although it is not illustrated, the sleeping position can be changed to the lateral position by forming additional chambers in order to support the thigh portion 15 (see Fig. 2B) in the pillow sheet 20 of Fig. 4B and then inflating the additional chambers in the pillow sheet 20.

Moreover, as can be seen from Fig. 4A or 4B, the pillow sheet 20 may have a base 21 which is provided under the pillow sheet body 20 contacted with a sheet (not shown). The base 21 allows the chambers 22 to be inflated in an upward direction of the pillow sheet body 20 contacted with a human body and is preferably made of a material capable of preventing the pillow sheet body 20 from sliding on the sheet.

Meanwhile, in Fig. 3, the pressure controller 30 controls pressure in each of the chambers 22 by actively supplying and discharging pressure to and from the chambers 22 so as to inflate and deflate the chambers 22.

Since the pressure controller 30 performs the pressure control on a chamber basis, the control can be actively carried out according to body position changes during sleep.

The pressure detection unit 40 detects pressure in each of the chambers 22 in real-time, thereby checking the chambers 22 pressed by a human body, detecting changes in the pressure applied to the chambers 22 during inhalation and exhalation of a user, and detecting vibration generated in a back part of a head due to the sleeping respiratory obstruction.

The storage unit 50 basically stores therein control data required for controlling the sleeping respiratory obstruction prevention apparatus. Further, the storage unit 50 stores therein optimal pressure pattern data obtained by making use of patterns of pressure changes in each of the chambers 22 during normal sleep in a supine position, i.e., during non-occurrence of the sleeping respiratory obstruction.

The artificial intelligence controller 60 loads the optimal pressure data from the storage unit 50 and checks whether or not a respiratory obstruction has occurred by comparing the loaded optimal pressure data with the pressure value for each of the chambers 22 received from the pressure detection unit 40. In case the occurrence of the respiratory obstruction has been checked, the artificial intelligent controller 60 outputs to the pressure controller 30 pressure control signal for controlling the inflation and the deflation of the chambers 22 to thereby treat the respiratory obstruction by guiding a body position of the user to a position capable of opening the upper airway 4 (see Fig. 2).

The data for controlling pressure in the chambers 22 allows the chambers 22 corresponding to the lower head portion 12 or the lower neck portion 14 to be comparatively inflated to thereby open the upper airway 4 of the user in a supine position, as illustrated in Fig. 2A.

The following is a specific description on how the artificial intelligence controller 60 checks whether or not the sleeping respiratory obstruction has occurred.

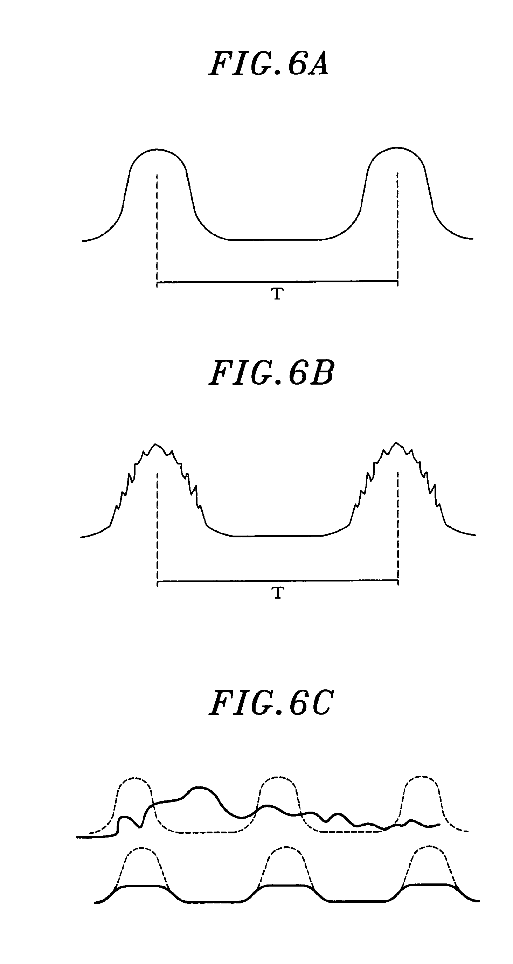

Figs. 6A, 6B and 6C illustrate breathing patterns of a user during sleep.

In the breathing pattern graphs of Figs. 6A, 6B and 6C, an X-axis indicates time, and a Y-axis indicate pressure in each of the chambers 22 detected by the pressure detection unit 40.

Increasing periods and decreasing periods in the graphs represent inhalation in which a user breathes in air through a mouth of a user and represent exhalation in which the user breathes out air through the mouth, respectively. Further, horizontal portions indicate pause periods between the inhalation and the exhalation.

Inhalation and exhalation patterns during normal sleep are measured on the chamber basis. Further, the storage unit 50 stores therein data such as graphs indicating pressure patterns in each of the chambers 22 and the like.

Since breath cycles T, patterns of pressure applied to each of the chambers 22 and the like vary depending on users, it is preferable to individualize the data such as graphs indicating pressure patterns in each of the chambers 22 and the like.

Whenever a user uses the sleeping respiratory obstruction prevention apparatus, the pressure detection unit 40 detects pressure in each of the chambers 22 of the pillow sheet 20 in real-time and, then, the detected data is compared with inhalation and exhalation patterns during normal sleep.

The following is a detailed description of the inhalation and the exhalation patterns during normal sleep and the data detected by the pressure detection unit 40 during a usage of the pillow sheet. Referring to Fig. 6A showing a normal sleep state, there is illustrated an approximately uniformly repetitive breath cycle T of inhalation and exhalation and uniform vibration waveforms. On the contrary, referring to Fig. 6B showing a state where a user is snoring, there is illustrated a repetitive breath cycle T of inhalation and exhalation which is similar to those in the normal sleep state and nonuniform vibration waveforms in the inhalation and the exhalation periods.

Therefore, when vibration waveforms that are nonuniform compared with those of the normal sleep state are appeared in the inhalation and the exhalation periods, it is determined that the user is snoring and, thus, a control for opening the upper airway 4 is initiated.

Further, when the user is suffering from the obstructive sleep apnea, there are shown in waveforms long pause periods instead of the repetitive breath cycles of inhalation and exhalation, or nonuniform inhalation and exhalation periods, or remarkably low fixed points in the inhalation periods, as illustrated in Fig. 6C. The dotted lines in Fig. 6C indicate vibration waveforms in the normal sleep state.

When there are shown in waveforms the long pause periods, or the nonuniform inhalation and exhalation periods, or the remarkably low fixed points in the inhalation periods compared with those of the normal sleep state, it is determined that the user is suffering from the obstructive sleep apnea and, thus, a control for opening the upper airway 4 is initiated.

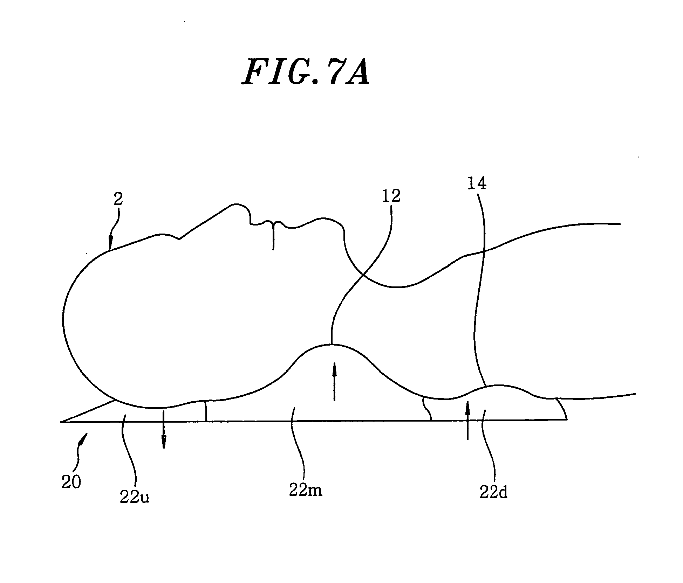

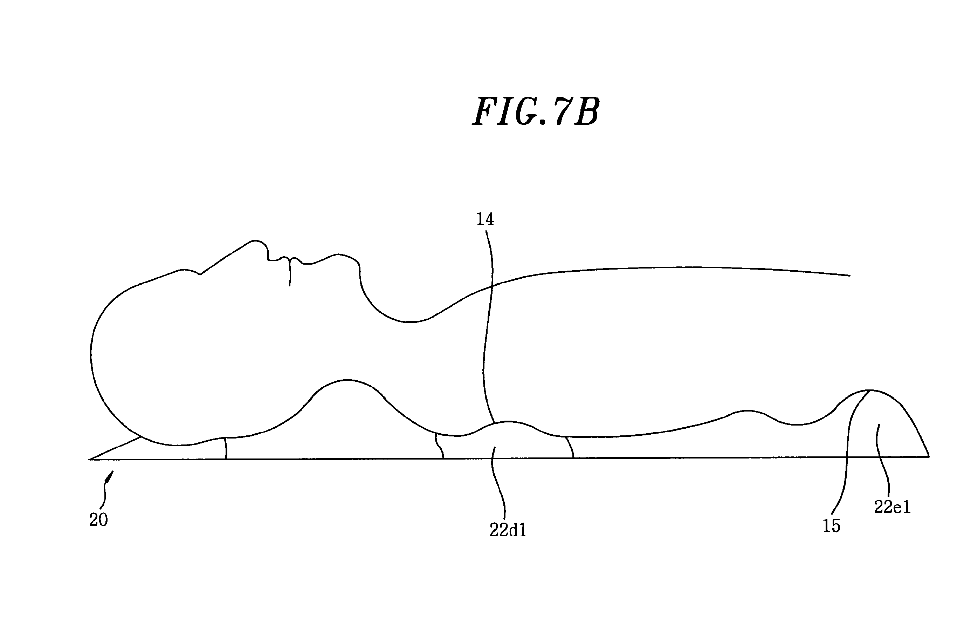

To do so, as shown in Fig. 7A, the artificial intelligence controller 60 controls the pressure controller 30 to apply pressure to the chambers 22m and 22d contacted with the lower head portion 12 and the lower neck portion 14 and discharge pressure from the chamber 22u contacted with an upper head portion to thereby raise the lower head portion 12 and the lower neck portion 14 and further to open the upper airway 4. As a result, the upper airway 4 is opened, and the snoring or the obstructive sleep apnea is treated.

In case the vibration waveforms of the user during sleep show the long pause periods, or the nonuniform inhalation and exhalation periods, or the remarkably low fixed points in inhalation periods compared with those in the normal sleep state even after the control of pressure in chambers, e.g., 22m, 22d and 22u, it is determined that the user is suffering from the obstructive sleep apnea and, thus, a control for opening the upper airway 4 is performed by changing a sleeping position to a lateral position.

To do so, the artificial intelligence controller 60 controls the pressure controller 30 to apply pressure to the chambers 22dl and 22el contacted with the lower neck portion 14 and one side of the thigh portion 15 to thereby raise the lower neck portion 14 and the one side of the thigh portion 15.

Accordingly, the sleeping position is guided to the lateral position and, also, the upper airway 4 is opened, which treats the snoring or the obstructive sleep apnea.

Preferably, the artificial intelligence controller 60 performs a fuzzy control by considering breath cycles of a user during a usage of a pillow sheet, heights of fixed points in inhalation periods, vibration waveforms and the like.

The fuzzy control is performed by processing boundary values as intermediate values based on a control operation designed to overcome hardware performance limits and by performing substantial control based on human decision and computation system using various input information from a variety of sensing units. Hence, the fuzzy control is suitable for checking whether or not a user is suffering from a sleeping respiratory obstruction.

In Fig. 3, the sleeping respiratory obstruction prevention apparatus may further include a power supply (not shown) for supplying power and the manipulation panel 28 for selecting functions of the sleeping respiratory obstruction prevention apparatus.

The power supply supplies electrical power to the pressure detection unit 40, the pressure controller 30, the storage unit 50 and the artificial intelligence controller 60. Although the power supply may be configured as an AC power supply, it is preferably configured to use a DC power supply such as a battery (not shown) to improve portability and remove uncomfortableness or dangerousness during sleep. Herein, the battery may be a liquid battery. Further, it may either be exchangeable or rechargeable.

As shown in Fig. 3, the manipulation panel 28 for interfacing with a user may have a number of keys or buttons as an input device for allowing the user to select an on/off operation and other functions such as a clock, a calculator and the like.

Moreover, the manipulation panel 28 may have a display device, e.g., an LED, an LCD or the like, for displaying an operation state of the sleeping respiratory obstruction prevention apparatus.

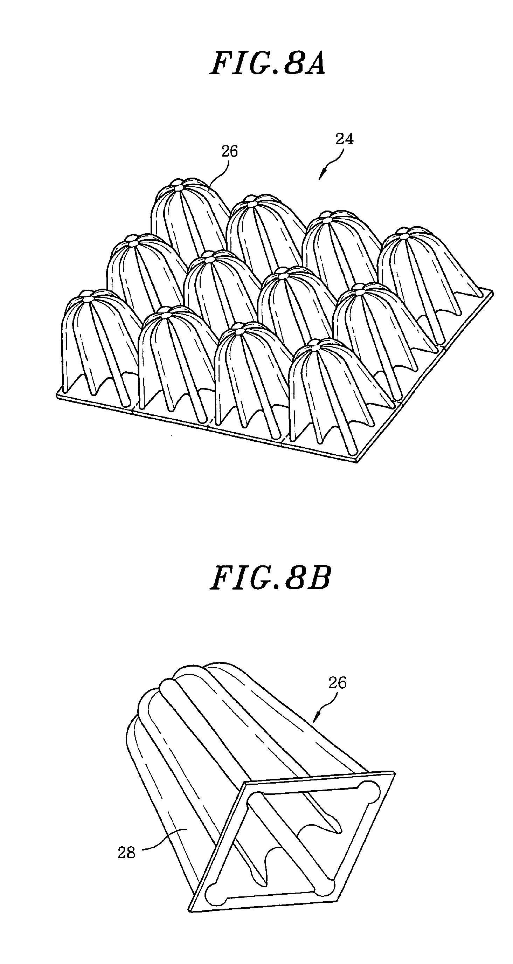

Figs. 8A and 8B illustrate perspective views of a chamber cell 24 and an air cell 26 in the chamber cell 24, wherein the chamber cell 24 constitutes one chamber. As shown, the chamber 22 has therein a plurality of air cells 26.

When pressure is supplied to the pillow sheet 20, the pressure is supplied to each of the chambers 22 and detected by the pressure detection unit 40. That is, the pressure is supplied to the chamber cells 24 or the air cells 26 in the chambers 24 and detected by the pressure detection unit 40.

Each air cell 26 is formed in a cup shape and has a plurality of column portions 28 to enhance elasticity, restoration force and strength.

The number of column portions 28 can be experimentally determined by considering internal pressure required for the air cell 26, a degree of inflation and a frequency of inflation. Fig. 8A illustrates the air cell 26 having eight columns 28, for example.

The number of air cells 26 contained in a single chamber cell 24 is determined by considering an area occupied by the chamber cell 24 and the like. For example, the chamber cell 24 illustrated in Fig. 8A has twelve air cells 26. Each of the air cells 26 may be formed to have a height required for a position thereof in the chamber cell 24.

Each of the air cells may be made of a material, e.g., a rubber, a PVC (polyvinyl chloride), or the like and a composite thereof. However, it will be apparent to those skilled in the art that the air cells may be varied in their number, arrangement and materials without being limited to the aforementioned examples.

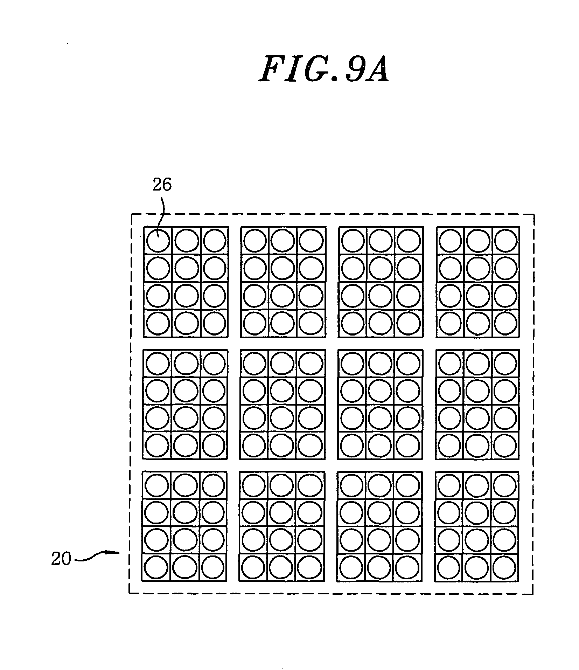

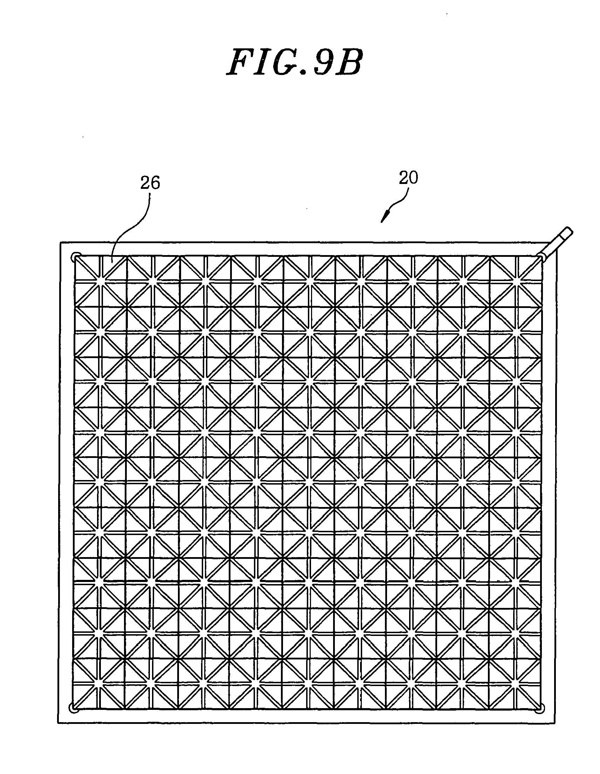

Figs. 9A and 9B provide examples of arranging the air cell 26 in the pillow sheet in accordance with the present invention.

As set forth above, one chamber cell 24 has therein a plurality of air cells 26 which are connected with each other in a regular pattern. Therefore, the air cells 26 are inflated and deflated while maintaining a predetermined shape thereof despite the pressure commonly applied to the air cells 26. Accordingly, it is possible to control pressure in the chamber cells 26 while maintaining a desired shape of the pillow sheet.

With the detection and the control of pressure in the air cells 26 in the chamber cells 24, when some of the air cells 26 are deflated by a load of a head contacted with the pillow sheet 20, other air cells 26 are inflated by pressure transmitted therefrom and, accordingly, the load on the air cells 24 can be distributed and controlled.

Although the sleeping respiratory obstruction is detected by the pressure detection unit 40, there may be provided other auxiliary devices, as illustrated in Fig. 3. For example, the auxiliary devices may include a sound sensor 42 and/or a vibration sensor 44 connected with the artificial intelligence controller 60.

In such a case, the storage unit 50 needs to further store therein optimal sound pattern data and/or optimal vibration variation data during normal sleep.

Whenever a user uses the sleeping respiratory obstruction prevention apparatus, the sound sensor 42 or the vibration sensor 44 detects a sound pattern or a vibration variation and, then, the detected data is compared with the optimal sound pattern data or the optimal vibration variation during normal sleep in the artificial intelligence controller 60.

That is, the artificial intelligence controller 60 loads the optimal sound pattern data or the optimal vibration variation data from the storage unit 50 and compares them with the sound pattern data or the vibration variation data received from the sound sensor or the vibration sensor 44. Accordingly, it is possible to check whether or not the sleeping respiratory obstruction has occurred in accordance with the present invention.

When sound pattern data or vibration variation data measured in real-time is deviated from an error range of the optimal sound pattern data or the optimal vibration variation data, the artificial intelligence controller 60 determines that the respiratory obstruction has occurred.

Since the respiratory obstruction can be checked by the sound sensor 42 or the vibration sensor 44 in addition to the pressure detection unit 40, the occurrence of the respiratory obstruction can be checked more accurately.

Besides, a blood oxygen saturation sensor 46 may be provided and connected with the artificial intelligence controller 60.

In such a case, the storage unit 50 further stores therein blood oxygen saturation data during normal sleep.

Whenever the user uses the pillow sheet, the blood oxygen saturation sensor 46 detects a blood oxygen saturation rate in real-time and, then, the detected data is provided to the artificial intelligence controller 60 where it is compared with optimal blood oxygen saturation data during normal sleep in the artificial intelligence controller 60.

More specifically, the blood oxygen saturation sensor 46 detects blood oxygen saturation data, which will then be sent to the artificial intelligence controller 60. In the artificial intelligence controller 60, the blood oxygen saturation data is compared with the optimal blood oxygen saturation data from the storage unit 50. Accordingly, it is also checked whether or not the respiratory obstruction has occurred.

When the blood oxygen saturation data measured in real-time is deviated from an error range of the optimal blood oxygen saturation data, the artificial intelligence controller 60 determines that the respiratory obstruction has occurred.

Since the sleeping respiratory obstruction can also be checked by the blood oxygen saturation sensor 46 in addition to the pressure detection unit 40, the sound sensor 42 and the vibration sensor 44, the occurrence of the sleeping respiratory obstruction can be checked more accurately.

However, in case a user frequently changes sleeping positions during sleep, the control effects of the artificial intelligence controller 60 may be reduced.

Therefore, when the user changes the sleeping positions during sleep, the sleeping position changes need to be detected to return the sleeping positions to a supine position or a lateral position capable of preventing the sleeping respiratory obstruction.

To do so, the artificial intelligence controller 60 detects whether or not the sleeping position has changed. When it is determined that the sleeping position is neither the supine position nor the lateral position, the artificial intelligence controller 60 performs the control for returning the sleeping position to the supine position or the lateral position.

In other words, when the user sleeps in the supine position without suffering from the sleeping respiratory obstruction, the pressure patterns in the chambers have symmetric breath cycles T and approximately uniform pressure levels in inhalation periods. Further, when the user sleeps in the lateral position without suffering from the sleeping respiratory obstruction, there are detected high pressure in chambers contacted with a face portion of the head and low pressure in chambers contacted with a back portion of the head. Therefore, when the changes in the pressure patterns are detected, the artificial intelligence controller 60 determines that the sleeping position of the user has changed and thus performs the control for changing the sleeping position of the user to the supine position or the lateral position capable of preventing the sleeping respiratory obstruction.

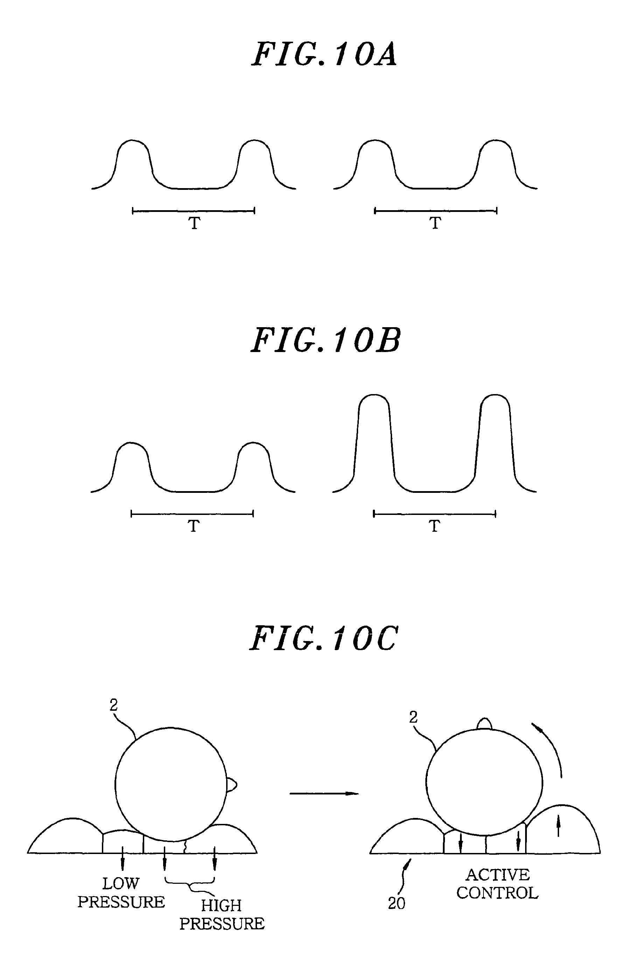

For example, when the user sleeps in the supine position without suffering from the sleeping respiratory obstruction, pressure patterns in right and left chambers have symmetric breath cycles T and approximately uniform pressure levels in inhalation periods, as illustrated in Fig. 10A. However, when the sleeping position of the user has changed, a pressure level of the pressure pattern in the right chambers becomes high, as shown in Fig. 10B.

In such a case, the artificial intelligence controller 60 determines that the sleeping position of the user has changed and thus performs the control for returning the sleeping position of the user to the supine position.

In order to return the sleeping position of the user to the supine position, pressure is supplied to the chambers contacted with the face portion of the head 2, whereas pressure is discharged from the chambers contacted with the back portion of the head 2, as shown in Fig. 10C. Such an asymmetric pressure distribution in the chambers 22 leads to an inclination of the pillow sheet 20, so that the sleeping position is changed by rotation.

The artificial intelligence controller 60 performs the same control when the pressure pattern changes the sleeping position from a lateral position capable of preventing the sleeping respiratory obstruction to another position.

Although it is not illustrated, when the user is sleeping in the lateral position without suffering from the sleeping respiratory obstruction, there are detected high pressure in chambers contacted with the face portion of the head due to a load and comparatively low pressure in chambers contacted with the back portions of the head. However, when the sleeping position of the user has changed, the pressure pattern also changes. In order to return the sleeping position of the user to the lateral position, pressure is supplied to the chambers positioned at one longitudinal end of the pillow sheet in the sleeping respiratory obstruction prevention apparatus, whereas pressure is discharged from the chambers positioned at the other end thereof. Such an asymmetric pressure distribution in the chambers leads to an inclination of the pillow sheet 20, so that the sleeping position is guided to the lateral position.

Meanwhile, as shown in Fig. 6B or 6C, in case a user has sudden body changes due to accidents or the like, it may not use the optimal pressure pattern data, the optimal sound pattern data, the optimal pressure variation data and the optimal blood oxygen saturation data, all being stored in the storage unit 50. Preferably, the manipulation panel 28 is configured to select a learning mode so that those data can be corrected.

When the learning mode is selected in the manipulation panel 28, the artificial intelligence controller 60 repetitively measures patterns of pressure changes in each of the chambers 22 during normal sleep and then stores the measured patterns in the storage as data for an artificial intelligence control.

Specifically, while a user is sleeping or stably lying on the pillow sheet 20 in a supine position for several minutes, a process for recording patterns of pressure applied to each of the chambers 22 is repeated multiple times at regular intervals.

Hence, previously stored data can be renewed and, thus, data reflecting body changes can be stored and utilized for the artificial intelligence control. Consequently, the sleeping respiratory obstruction can be checked more accurately.

In case a single pillow sheet is used by various users, the previous data needs to be prevented from being terminated by an input of new data.

Therefore, the manipulation panel 28 is preferably configured to have a user selection mode.

Further, the storage unit 50 is preferably configured to store therein in advance data containing optimal pressure pattern data obtained by making use of patterns of pressure changes in each of the chambers 22 during normal sleep in a supine position on a user basis.

Furthermore, when the user selection mode and a user are selected, it is preferable that the artificial intelligence controller 60 be configured to load data of the selected user.

Accordingly, the data used by a previous user can be stored even in case a user who is not a pillow sheet owner of the sleeping respiratory obstruction apparatus uses a pillow sheet or in case a single pillow sheet of the sleeping respiratory obstruction apparatus is commonly used by various users. As a result, the pillow sheet can be used by various users.

### (Second Embodiment)

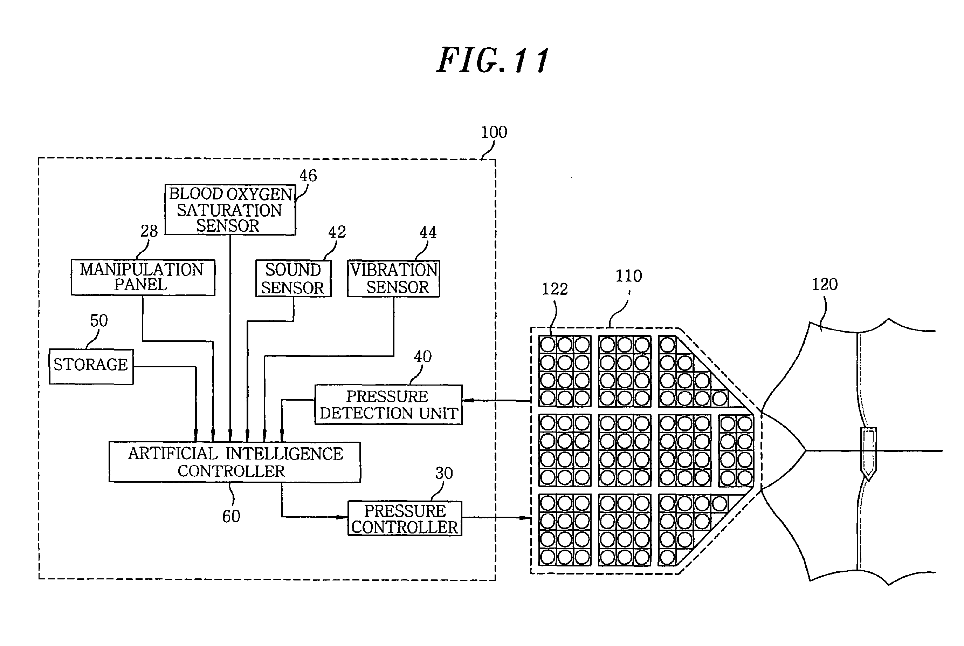

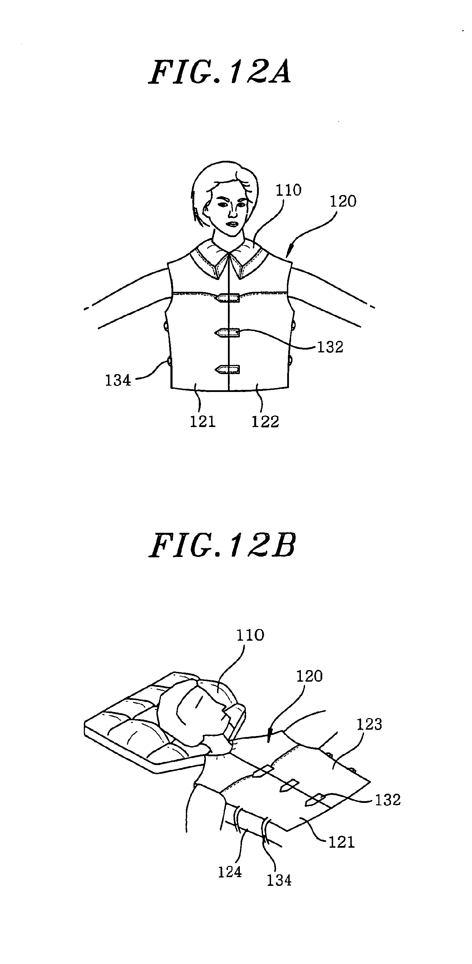

Fig. 11 offers a block diagram of an apparatus for preventing a sleeping respiratory obstruction in accordance with a second embodiment of the present invention; and Fig. 12 shows an operation state of using the sleeping respiratory obstruction prevention apparatus shown in Fig. 11.

As shown in Fig. 11, the sleeping respiratory obstruction prevention apparatus includes a pillow sheet 110, a wearable unit 120 and a control module 100. The sleeping respiratory obstruction prevention apparatus of the second embodiment of the present invention is different from that of the first embodiment in that the second embodiment further includes the wearable unit 120 and the wearable unit 120 is coupled to the pillow sheet 110. Therefore, a detailed description for the same components through the drawings will be omitted for the sake of simplicity.

The wearable unit 120 is designed to be wearable by considering wearability and usability of a unit to be put on a human body. The wearable design requires a functional structure and a proper material selection.

In particular, it is required to select a material capable of managing elements harmful to a human body and providing comfortableness, safety, electromagnetic wave shielding effects, insulation and the like.

The wearable unit 120 needs to have openings in all parts of the body or under the arm where perspiration is generally profuse to thereby eliminate the perspiration and control a body temperature during sleep. Such openings enable heat or moisture to be quickly removed from a garment, which improves the comfortableness. Further, a sawing technique needs to minimize a friction between a material and a human body.

Accordingly, the wearable unit 120 may be made of a health-oriented material such as a chitosan fiber, a silver fiber, a bamboo fiber or the like, a high-tech material such as Aquatrans^{™}, Coolmax^{R} mesh or the like, or a environmentally friendly material such as an organic cotton, Tencel, a natural mineral ion textile or the like, for example.

The pillow sheet 110 is coupled with the wearable unit 120. Therefore, the pillow sheet 110 supports the head and the neck of the user when the user sleeps while wearing the wearable unit 120, thereby preventing the sleeping respiratory obstruction such as snoring, obstructive sleep apnea and the like.

According to the present invention, the pillow sheet 110 serves as a collar of the wearable unit 120. Preferably, the pillow sheet 110 can be used by unfolding the collar.

In case the pillow sheet 110 is not used to support the head and the neck of the user as described above in order for the pillow sheet 110 to serve as the collar of the wearable unit 120 while being coupled with the wearable unit 120, the pillow sheet 110 may be air-inflated by a supply of air pressure.

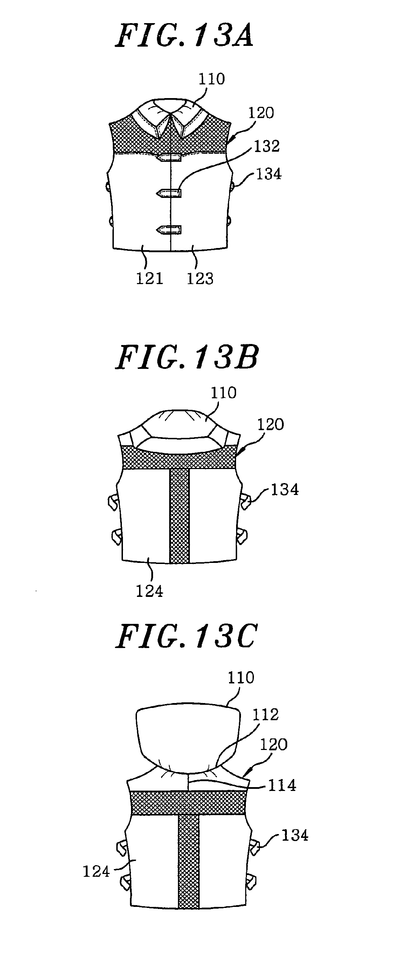

For example, as shown in Fig. 13C, the collar is designed to have a large size so as not to be turned over behind the neck when the pressure is released in case of a narrow color in its width. Moreover, by injecting the pressure only through a shirred/darted part 112, a shape of the collar can be maintained even when the user moves while wearing the wearable unit 120.

Further, a narrow yoke 114 is formed under the neck in the back of the wearable unit 120 so that air can be inflated even when the collar is turned over.

Before the pressure is supplied to the pillow sheet 110 as illustrated in Fig. 12A, the pillow sheet 110 is coupled as a collar with the wearable unit 120 in a state of being deflated. However, after the pressure is supplied to the pillow sheet 110 for sleeping as shown in Fig. 12B, the pillow sheet 110 is inflated and thus serves as a pillow.

The pillow sheet 110 does not need to be constantly coupled with the wearable unit 120. In other words, the pillow sheet 110 is attached to the wearable unit 120 only when the user sleeps and detached therefrom in other cases. Preferably, the pillow sheet 110 is attached to and detached from the wearable unit 120 by a coupling member such as a zipper (not shown) or a velcro (not shown).

Although it is not illustrated, the wearable unit 120 may be a winter jacket or jumper. However, since it is worn during sleep, a vest as shown in Figs. 12A and 12B is preferred.

The vest is a garment having no sleeves, so that it has good permeability compared with a garment having sleeves and prevents a body temperature from increasing during sleep.

Further, although it is not illustrated, such vest may have various modified examples, e.g., one with a front closure, one with a front closure and open sides, one with neither a closure nor an open side, and the like. Herein, the vest with a front closure and open sides will be described.

Figs. 13A to 13C illustrate a front view and rear views of a modified example of the wearable unit shown in Fig. 11.

As illustrated in Figs. 13A to 13C, the wearable unit 120 with a front closure and open sides includes front parts 121 and 123 for covering a front surface of a human body and a rear part 124 covering a rear surface of the human body. The front parts 121 and 123 include a front right part 121 positioned at a right side of the front surface and a front left part 123 positioned at a left side of the front surface in view of a user wearing the wearable unit 120.

The wearable unit 120 can be worn while the front closure and the open sides are being opened. Since the wearable unit 120 can be conveniently put on and taken off, it can be properly used by a patient who has a difficulty in moving.

Further, the front closure and the open sides facilitate the permeability, so that the body temperature can be prevented from increasing during sleep. Moreover, the wearable unit 120 can be worn regardless of a body size and thus does not make the user feel uncomfortable during sleep. In this way, sleep disturbing factors can be minimized.

After the wearable unit 120 is put on a human body, the front closure between the front right side 121 and the front left side 123 is fixed by fasteners 132 and, also, the open sides between the front parts 121 and 123 and the rear part 124 are fixed by fasteners 134, resulting in convenient usage. Further, the wearable unit 120 can be comfortably worn by controlling a width thereof.

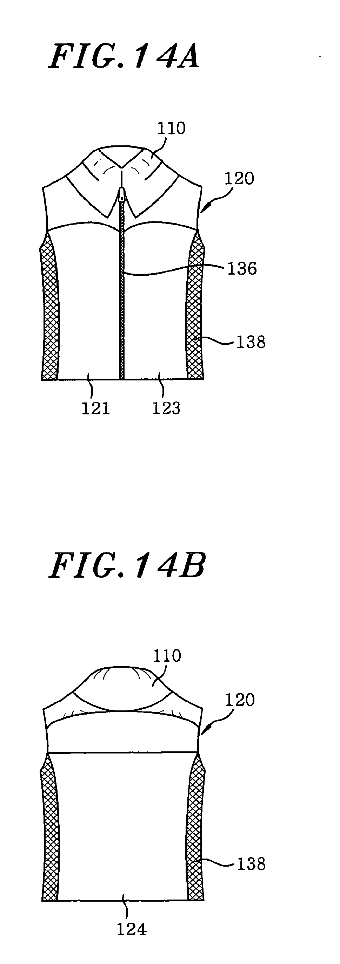

Referring to Figs. 14A and 14B, there is illustrated a front view and a rear view of another example of the wearable unit shown in Fig. 11.

As illustrated in Figs. 14A and 14B, it is also possible to couple the front right part 121 and the front left part 123 by a zipper 136, instead of the fasteners 132 and 134, and couple the front parts 121 and 123 and the rear part 124 by rubber bands 138.

Therefore, a position of the wearable unit 120 can be adjusted according to an opening degree of the zipper 136. Hence, even the pillow sheet 110 with the wearable unit 120 is made to have a small size, it can be reasonably used for preventing a sleeping respiratory obstruction.

Moreover, the front parts 121 and 123 are not separated from a rear part 124, which provides the comfortableness. Further, it is possible to minimize uncomfortableness during sleep by replacing the rubber bands 38 with an elastic material having good permeability.

Although it is not illustrated, it is possible to couple the front right part 121 and the front left part 123 by fasteners and couple the front parts 121 and 123 and the rear part 124 by zippers. Further, it is also possible to couple the front right part 121 and the front left part 123 by a zipper and couple the front parts 121 and 123 and the rear part 124 by zippers.

Meanwhile, referring back to Fig. 11, the control module 100 includes a pressure controller 30, a pressure detection unit 40, a storage unit 50, and an artificial intelligence controller 60, a sound sensor 42, a vibration sensor 44, a blood oxygen saturation sensor 46 and an manipulation panel 28, all of which are substantially same as those in the first embodiment and, therefore, a detailed description therefor will be omitted.

As described in the first embodiment of the present invention, the artificial intelligence controller 60 loads the optimal pressure pattern data from the storage unit 50 and checks whether or not a respiratory obstruction occurs by comparing the loaded optimal pressure pattern data with the pressure in each of the chambers 122 received from the pressure detection unit 240. In case the occurrence of the respiratory obstruction has been checked, the artificial intelligent controller 60 outputs pressure control signal for controlling an inflation/deflation of the chambers 122 to the pressure controller 30 so that the respiratory obstruction can be treated with a body position enabling an upper airway of a pillow user to be opened.

Herein, the data for controlling pressure in the chambers 122 to open the upper airway of the pillow user allows the chambers 122 corresponding to portions under the head or under the neck to be comparatively inflated in a supine position or makes a user to change a sleeping position to a lateral position.

Accordingly, the sleeping respiratory obstruction prevention apparatus with the wearable unit 120 can prevent and treat an obstructive sleep apnea caused by repetitive closure of an upper airway in a neck of a body and a habitual snoring related to the obstructive sleep apnea.

While the invention has been shown and described with respect to the embodiments, it will be understood by those skilled in the art that various changes and modification may be made without departing from the scope of the invention as defined in the following claims.

## Claims

1. An apparatus for preventing a sleeping respiratory obstruction, comprising:
a pillow sheet having a plurality of chambers on which a body of a user is supported; and
a control module for controlling inflation and deflation of the chambers supporting the body of the user in order to open an upper airway of the user being subjected to the sleep respiratory obstruction during sleep.

2. The apparatus of claim 1, wherein the control module includes:
a pressure controller for supplying and discharging pressure to and from the respective chambers for the inflation and the deflation of the respective chambers;
a pressure detection unit for detecting the pressure in each of the chambers;
a storage unit for storing optimal pressure pattern data obtained by making use of patterns of pressure changes in each of the chambers during normal sleep; and
an artificial intelligence controller for comparing the pressure pattern data with the pressure in each of the chambers received from the pressure detection unit to check whether or not the sleep respiratory obstruction has occurred, and providing to the pressure controller, in case the occurrence of the sleep respiratory obstruction has been detected, pressure control signal for controlling the inflation and the deflation of the chambers so that the upper airway of the user is made open to thereby treat the respiratory obstruction.

3. An apparatus for preventing a sleeping respiratory obstruction, comprising:
a pillow sheet having a plurality of chambers on which a body of a user is arranged;
a pressure controller for supplying and discharging pressure to and from the respective chambers for an inflation and a deflation of the respective chambers;
a pressure detection unit for detecting pressure in each of the chambers;
a storage unit for storing optimal pressure pattern data obtained by making use of patterns of pressure changes in each of the chambers during normal sleep; and
an artificial intelligence controller for comparing the pressure pattern data with the pressure in each of the chambers received from the pressure detection unit to check whether or not a respiratory obstruction has occurred, and providing to the pressure controller, in case the occurrence of the respiratory obstruction has been detected, pressure control signal for controlling the inflation and the deflation of the chambers so that an upper airway of the user is made open.

4. An apparatus for preventing a sleeping respiratory obstruction, comprising:
a pillow sheet having a plurality of chambers on which a body of a user is arranged;
a wearable unit detachably coupled to the pillow sheet and be worn on the body during sleep;
a pressure controller for supplying and discharging pressure to and from the respective chambers for an inflation and a deflation of the respective chambers;
a pressure detection unit for detecting pressure in each of the chambers;
a storage unit for storing optimal pressure pattern data obtained by making use of patterns of pressure changes in each of the chambers during normal sleep; and
an artificial intelligence controller for comparing the pressure pattern data with the pressure in each of the chambers received from the pressure detection unit to check whether or not a respiratory obstruction has occurred, and providing to the pressure controller, in case the occurrence of the respiratory obstruction has been detected, pressure control signal for controlling the inflation and the deflation of the chambers to support a head and a neck of the user so that an upper airway of the user is made open to thereby treat the respiratory obstruction.

5. The apparatus of claim 2 or 3, wherein the pressure control signal controls the inflation and the deflation of the chambers supporting a head and a neck of the user during sleep so that the upper airway of the user is made open.

6. The apparatus of claim 2 or 3, wherein the pressure control signal controls the inflation and the deflation of the chambers to support a thigh portion of the user during sleep so that a sleeping position of the user is changed into a lateral position by rotation, to thereby open the upper airway of the user.

7. The apparatus of any one of claims 2 to 4, wherein each of the chambers includes a chamber cell, the chamber cell having therein a plurality of air cells, and wherein the pressure to and from the chambers is supplied for an inflation and a deflation of each of the air cells.

8. The apparatus of any one of claims 2 to 4, wherein the pillow sheet has on a bottom surface thereof a base for supporting the inflation of the chambers and preventing a sliding of the pillow sheet.

9. The apparatus of claim 7, wherein the pressure in each of the air cells is detected by the pressure detection unit and then provided to the artificial intelligence controller.

10. The apparatus of any one of claims 2 to 4, further comprising a sound sensor connected with the artificial intelligence controller, for detecting sound pattern data of the user during sleep,
wherein the storage unit further stores therein optimal sound pattern data during normal sleep, and
wherein the artificial intelligence controller compares the optimal sound pattern data with the sound pattern data detected by the sound sensor to thereby check whether or not the respiratory obstruction has occurred.

11. The apparatus of any one of claims 2 to 4, further comprising a vibration sensor connected with the artificial intelligence controller, for detecting vibration variation data of the user during sleep,
wherein the storage unit further stores therein optimal vibration variation data during normal sleep, and
wherein the artificial intelligence controller the optimal vibration variation data compares the optimal vibration variation data with the vibration variation data detected by the vibration sensor to thereby check whether or not the respiratory obstruction has occurred.

12. The apparatus of any one of claims 2 to 4, further comprising a blood oxygen saturation sensor connected with the artificial intelligence controller, for detecting a blood oxygen saturation data of the user during sleep,
wherein the storage unit further stores therein optimal blood oxygen saturation data during normal sleep, and
wherein the artificial intelligence controller compares the optimal blood oxygen saturation data with the blood oxygen saturation data detected by the blood oxygen saturation sensor to thereby detect whether or not the respiratory obstruction has occurred.

13. The apparatus of any one of claims 1 to 12, further comprising:
a power supply for supplying electrical power to the apparatus; and
a manipulation panel for allowing a user to select an on/off operation of the apparatus.

14. The apparatus of claim 13, wherein the manipulation panel is configured to select an operation mode of the apparatus on a user basis; and
wherein the artificial intelligence controller compares the sound pattern data, the vibration variation data or the blood oxygen saturation data detected by the sound sensor, the vibration sensor or the blood oxygen saturation sensor for a selected user with the optimal sound pattern data, the optimal vibration variation data or the optimal blood oxygen saturation data, respectively, to thereby check whether or not the respiratory obstruction has occurred.

15. The apparatus of claim 13, wherein the manipulation panel is configured to select a learning mode, and in case the learning mode is selected, patterns of pressure changes during normal sleep are repetitively measured on a chamber basis and then stored in the storage unit.

16. The apparatus of claim 14, wherein the artificial intelligence controller provides the pressure control signal to the pressure controller by performing a fuzzy control.

17. The apparatus of claim 1, further comprising:
a wearable unit detachably coupled to the pillow sheet and be worn on the user during sleep.

18. The apparatus of claim 4 or 17, wherein the pillow sheet serves as a collar of the wearable unit when the pillow sheet is not used to support the head and the neck of the user and is usable when the collar is unfolded.

19. The apparatus of claim 18, wherein the pillow sheet includes an attaching/detaching unit for attaching/detaching the pillow to/from the wearable unit.

20. The apparatus of claim 19, wherein the attaching/detaching units includes a zipper or a Velcro.

21. The apparatus of claim 4 or 17, wherein the wearable unit is formed as a vest.

22. The apparatus of claim 21, wherein the wearable unit is worn with a fastener or a zipper.

23. The apparatus of claim 22, wherein the wearable unit includes:
a front right part positioned at a right side of a front surface of the body;
a front left part positioned at a left side of the front surface of the body; and
a rear part positioned on a rear surface of the body in view of a user wearing the wearable unit,
wherein the fastener or the zipper is provided between the front left part and the front right part, between the front left part and the rear part and between the front right part and the rear part.
